(19) Europäisches Patentamt
European Patent Office
Office européen des brevets

(11) Publication number: **0 531 958 A1**

(12) **EUROPEAN PATENT APPLICATION**

(21) Application number: **92115387.0**

(22) Date of filing: **09.09.92**

(51) Int. Cl.[5]: **C07D 215/56**, A61K 31/40, C07D 401/04, C07D 471/04, C07D 401/12

(30) Priority: **13.09.91 IT MI912426**

(43) Date of publication of application: **17.03.93 Bulletin 93/11**

(84) Designated Contracting States: **AT BE CH DE DK ES FR GB GR IE IT LI LU MC NL PT SE**

(71) Applicant: **MEDIOLANUM FARMACEUTICI S.P.A.**
**Via San Giuseppe Cottolengo 31**
**I-20143 Milano(IT)**

(72) Inventor: **Cecchetti, Violetta**
**Via Piervittori, 6**
**I-06100 Perugia(IT)**
Inventor: **Fravolini, Arnaldo**
**Strada Gualtarella, 7/d**
**I-06080 S. Sisto (Perugia)(IT)**
Inventor: **Terni, Patrizia**
**V. Sazzan, 9**
**I-20146 Milan(IT)**
Inventor: **Pagella, Pier Giuseppe**
**Isola Sant'Antonio**
**I-15050 Frazione Catraglia (Alessandria)(IT)**
Inventor: **Tabarrini, Oriana**
**Via Eucubina 48**
**I-06100 Perugia(IT)**

(74) Representative: **Gervasi, Gemma, Dr. et al**
**NOTARBARTOLO & GERVASI Srl 33, Viale Bianca Maria**
**I-20122 Milano (IT)**

(54) **6-Aminoquinolones, their synthesis and their use as antibacterial agents.**

(57) 6-aminoquinolones, fit for being used as antibacterial agents, having the following general formula:

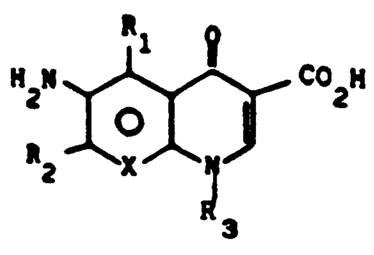

(I)

wherein
X = CH, $CCH_3$, CF, N;
$R_1$ = H, F, $NH_2$;
$R_2$ =

EP 0 531 958 A1

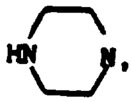, 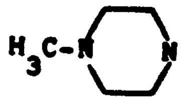, 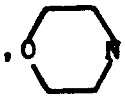, 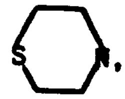, 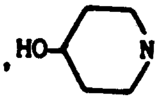

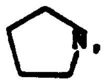, 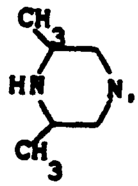, 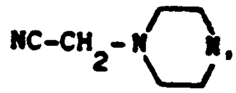, 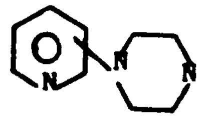

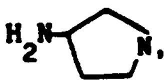, 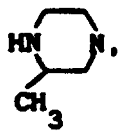, 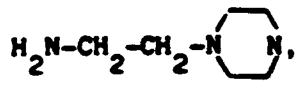,

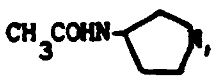, 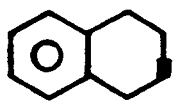, 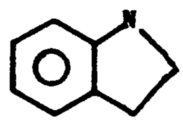

$R_3$ =

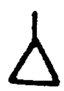, t-butyl, 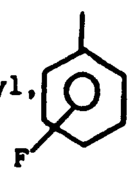.

THE PRIOR ART:

Fluoroquinolones having a substituting F atom in C-6 are known and it is known as well their use as antibacterial agents.

Preparation and features of said quinolones are described for instance in the following patents: DE 3142854 (Bayer AG), EP 58614 (Dainippon Farm), EP 287951 (Otsuka Pharm.) and in the articles J. Med. Chem. 28, 1558 (1985) and J. Med. Chem 32, 537 (1989).

Said fluoroquinolones exert a fair antibacterial activity, but show the drawback of easily passing through the blood-brain barrier.

Summary:

A new class of quinolones was now found which allows to overcome the drawbacks of the prior art. The quinolones of the present invention are characterized in that they have an amino group in the C-6 position and have the following general formula (I):

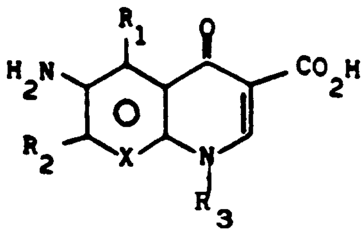

(I)

wherein

X = CH, $CCH_3$, CF, N;

$R_1$ = H, F, $NH_2$;

$R_2$ =

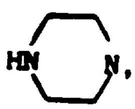 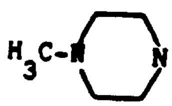 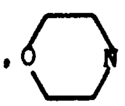 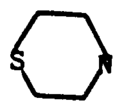 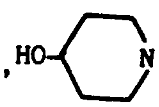

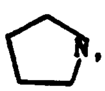 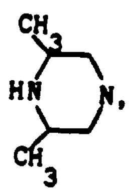 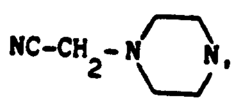 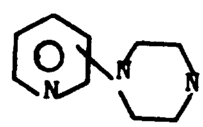

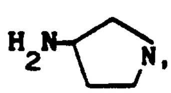 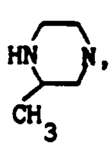 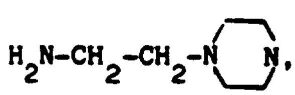

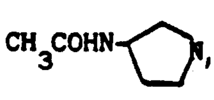 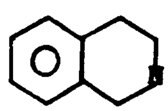, 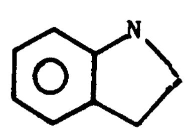

$R_3$ =

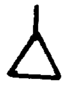 , t-butyl, 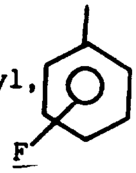

Said aminoquinolones have a considerable antibacterial activity and furtherly show, with respect to the fluoroquinolones of the prior art, a higher solubility in water and produce smaller side effects at the level of the central nervous system, as they pass with greater difficulty through the blood-brain barrier.

DETAILED DESCRIPTION OF THE INVENTION:

The features and advantages of the 6-aminoquinolones of the present invention, as well as the methods for their preparation, will be better illustrated during the following detailed description. The 6-aminoquinolones according to the invention have the following general formula (I):

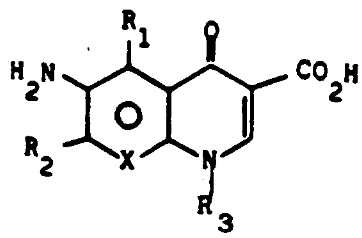

(I)

wherein

X = CH, $CCH_3$, CF, N;

$R_1$ = H, F, $NH_2$;

$R_2$ =

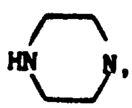, 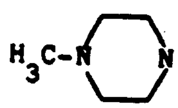, 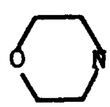, 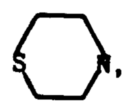, 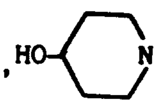

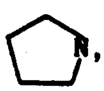, 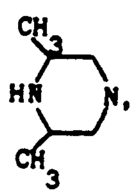, 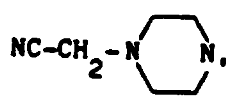, 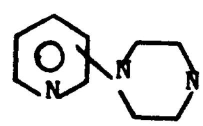

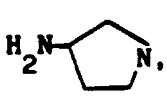, 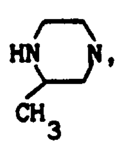, 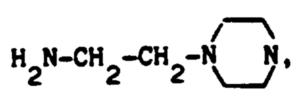,

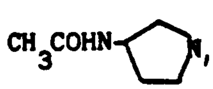, 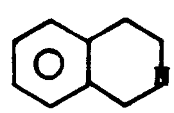, 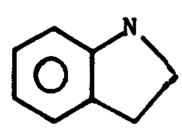

$R_3$ =

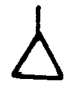 , t-butyl, 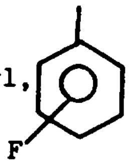

The compound having general formula (I) in which X = CH, $R_1$ = H, $R_2$ =

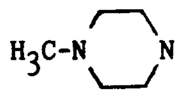

and $R_3$ = cyclopropyl, can be prepared according to the following steps:

step a) 2,4-dichloro-5-nitrobenzoic acid of formula (2):

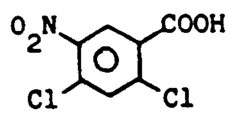

(2)

is made to react with an eccess of thionyl chloride under reflux conditions and the chloride thus obtained is made to react with ethyl $\beta$-dimethylamino acrylate and triethylamine in toluene at a temperature from 85 to 95 °C. The molar ratio between ethyl $\beta$-dimethylamino acrylate and 2,4-dichloro-5-nitrobenzoic acid is between 1:1 and 1:1.2;

step b) the ethyl 2-(2,4-dichloro-5-nitrobenzoyl)-3-dimethylamino acrylate (3), obtained according to step a), is made to react with cyclopropylamine in ethanol and ethyl ether under ice cooling. The molar ratio between compound (3) and cyclopropylamine is between 1:1.4 and 1:1.6;

step c) the ethyl 2-(2,4-dichloro-5-nitrobenzoyl)-3-cyclopropylamino-acrylate (4), obtained according to step b), is made to react with $K_2CO_3$ in dimethylformamide (DMF) under reflux conditions;

step d) the ethyl 1-cyclopropyl-7-chloro-6-nitro-1,4-dihydro-4-oxo-quinoline carboxylate (5), obtained according to step c), is made to react with N-methylpiperazine in DMF, at a temperature between 100 and 110 °C. The molar ratio between compound (5) and N-methylpiperazine is between 1:1 and 1:2;

step e) the ethyl 1-cyclopropyl-6-nitro-7-(4-methylpiperazinyl)-1,4-dihydro-4-oxo-quinoline carboxylate (6), obtained according to step d), dissolved in 2-methoxyethanol, is reduced by means of Raney nickel in a stream of $H_2$ at room temperature;

step f) the ethyl 6-amino-1-cyclopropyl-7-(4-methyl-1-piperazinyl)-1,4-dihydro-4-oxo-quinoline carboxylate (7), obtained according to step e), is hydrolyzed by means of a reflux treatment with a 1N solution of NaOH, followed by a pH adjustment (pH = 7), by addition of a HCl solution at room temperature.

A compound is thus obtained having general formula (I) where X = CH, $R_1$ = H, $R_2$ =

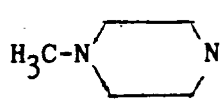

and $R_3$ = cyclopropyl. By working according to the same method, while using proper reactants, namely piperazine and derivatives thereof and pyrrolidine and derivatives thereof, in replacement of N-methylpiperazine, there are obtained many other compounds falling under the same general formula (I), some of which will be described in the examples hereinbelow.

The compound having general formula (I), with X = CF, $R_1$ = H, $R_2$ =

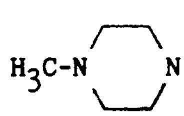

and $R_3$ = cyclopropyl, can be prepared according to the following steps:

step a) 2,3,4,-trifluoronitrobenzene is made to react with N-methylpiperazine in toluene at room temperature, with a molar ratio between the two reactants of 1:1;

step b) the 3,4-difluoro-2(4-methyl-1-piperazinyl)nitrobenzene (33), obtained according to step a), is made to react with cyclopropylamine in hexamethylphosphoric triamide (HMPA) at 40-60 °C and with an eccess of said reactant;

step c) the 4-cyclopropylamino-3-fluoro-2-(4-methyl-1-piperazinyl) nitrobenzene (34), obtained according to step b), is made to react with diethylethoxymethylenmalonate at 130-150 °C in the presence of an eccess of this reactant;

step d) the ethyl N-cyclopropyl-N-[2-fluoro-4-nitro-3-(4-methyl-1-piperazinyl)benzene] aminomethylenemalonate (35), obtained according to step c), is made to react with an eccess of polyphosphoric acid at 85-95 °C;

step e) the ethyl 1-cyclopropyl-8-fluoro-6-nitro-7-(4-methyl-1-piperazinyl)-1,4-dihydro-4-oxo-quinoline carboxylate (36), obtained according to step d), is made to react with iron powder at 40-50 °C;

step f) the ethyl 6-amino-1-cyclopropyl-8-fluoro-7-(4-methyl-1-piperazinyl)-1,4-dihydro-4-oxo-quinoline carboxylate (37), obtained according to step e), is made to react with a 1N solution of NaOH under reflux conditions. There is thus obtained a compound having general formula (I), in which X = CF, $R_1$ = H, $R_2$ =

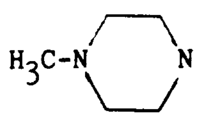

and $R_3$ = cyclopropyl.

By slightly modifying the methods hereinabove, it is possible to obtain the different compounds having general formula (I); the preparation of a few of such compounds is described in the following examples.

Such examples, concerning the preparation of compounds according to the invention, are supplied for merely illustrative purposes but do not limit in any way the scope of the invention.

EXAMPLE 1

a) Preparation of compound (3):

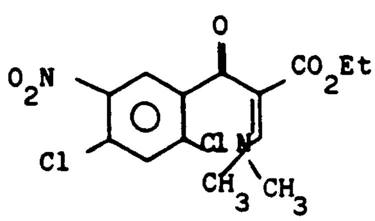

(3)

5 g (0.021 mol) of 2,4-dichloro-5-nitrobenzoic acid (2) are suspended in 20 ml of thionyl chloride and then are refluxed for 3 h. The reaction mixture is dried out under vacuum, anhydrous benzene is added to the residual oil and the mixture is dried out once more; the operation is repeated twice. Then 50 ml of dry toluene, 3.2 g (0.022 mol) of ethyl β-dimethylamino acrylate and 3 g of triethylamine (0.03 mol) are added to the residue. The reaction mixture is kept at 90 °C for 1.5 h. The thus formed solid is filtered and the solution is dried out under vacuum. The residual oil is chromatographed on silica gel, eluting with gradually increasing amounts of ethyl acetate/cyclohexane. There are thus obtained 2.1 g (yield = 42. 2%) of ethyl 2-(2,4-dichloro-5-nitrobenzoyl)-3-dimethylaminoacrylate (3) in the form of an oil. $^1$H-NMR ($CDCl_3$): 1.00 (3H, t, J = Hz, $CH_2CH_3$), 3.00 and 3.40 (3H each, br s, $NCH_3$), 3.95 (2H, q, J = 7 Hz, $CH_2CH_3$), 7.50 (1H, s, H-3), 7.85 (2H, s, vinylic H and H-6).

b) Preparation of compound (4):

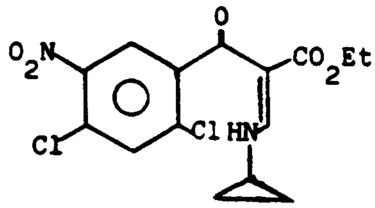

(4)

0.0722 g (0.002 mol) of compound (3) are suspended in 2.5 ml of ethanol and there is then added ethyl ether up to dissolution. To the ice cooled solution there are added 0.175 g (0.003 mol) of cyclopropylamine. The mixture is then brought to room temperature and after 15 minutes 0.710 g of a precipitate are collected consisting of ethyl 2-(2,4-dichloro-5-nitrobenzoyl)-3-cyclopropylamino acrylate (4). Yield = 88.7%; m.p. = 120-122 ° C.
$^1$H-NMR ($CDCl_3$): 0.75-1.15 (7H, m, $CH_2CH_3$ and cyclopropyl $CH_2$), 2.75-3.10 (1H, m, cyclopropyl CH), 3.90 (2H, q, J = 7 Hz, $CH_2CH_3$), 7.40 (1H, s, H-3), 7.65 (1H, s, H-6), 8.15 (1H, d, J = 12 Hz, vinyl H), 10.80-11.00 (1H, m, NH).

c) Preparation of compound (5):

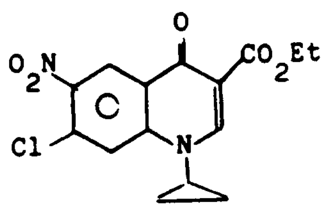

(5)

0.5 g (0.0013 mol) of compound (4) are dissolved in 8 ml of DMF and 0.200 g of $K_2CO_3$ are then added. The reaction mixture is refluxed for 1 h. After cooling, the precipitated solid is collected by means of a (vacuum) pump and washed with $H_2O$. It consists of ethyl 1-cyclopropyl-7-chloro-6-nitro-1,4-dihydro-4-oxo-quinoline carboxylate (5). Yield = 71.5%; m.p. = 255-257 ° C.
$^1$H-NMR (TFA/DMSO-$d_6$): 1.30-1.75 (7H, m, $CH_2CH_3$ and cyclopropyl $CH_2$), 4.10-4.40 (1H, m, cyclopropyl CH), 4.70 (2H, q, J = 7 Hz, $CH_2CH_3$), 8.85 (1H, s, H-8), 9.10 (1H, s, H-5), 9.40 (1H, s, H-2).

d) Preparation of compound (6):

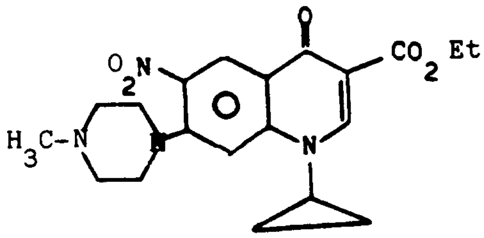

(6)

0.170 g (0.0005 mol) of compound (5) are dissolved in 7 ml of DMF and 0.100 g of N-methylpiperazine are then added. The mixture is heated at 100-110 ° C for 1 h. The solvent is removed under vacuum, there are added 10 ml of EtOH and the precipitated solid is filtered, washed with water and crystallized from DMF (m.p. = 218-221 ° C). Said solid consists of ethyl 1-cyclopropyl-6-nitro-7-(4-methylpiperazinyl)-1,4-dihydro-4-oxo-quinoline carboxylate (6). Yield = 86.2%.

$^1$H-NMR (TFA): 1.30-1.80 (7H, m, $CH_2CH_3$ and cyclopropyl $CH_2$), 3.15 (3H, br s, CH), 3.50-4.30 (9H, m, piperazine H and cyclopropyl CH), 4.65 (2H, q, J = 7 Hz, $CH_2CH_3$), 8.05 (1H, s, H-8), 9.10 (1H, s, H-5), 9.30 (1H, s, H-2).

e) Preparation of compound (7):

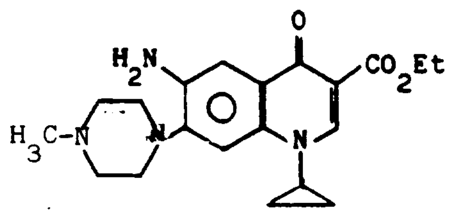

(7)

1.3 g of the ester (6) are dissolved in 100 ml of 2-methoxyethanol at a relatively high temperature. The solution is then brought to room temperature, there is added Raney nickel and a stream of $H_2$ is allowed to flow for 3.5 h. The solution is filtered and then concentrated to one third of the volume. The precipitated white solid is washed first with water and then with ethanol. There is obtained ethyl 6-amino-1-cyclopropyl-7-(4-methyl-1-piperazinyl)-1,4-dihydro-4-oxo-quinoline carboxylate (7), with a yield of 75% and a m.p. = 282-285 ° C.

$^1$H-NMR (TFA): 1.25-1.75 (7H, m, cyclopropyl $CH_2CH_3$ and $CH_2$), 3.20 (3H, br s, NCH), 3.55-4.05 (1H, m, cyclopropyl CH), 4.75 (2H, q, J = 7 Hz, $CH_2CH_3$), 8.45 (1H, s, H-8), 8.75 (1H, s, H-5), 9.35 (1H, s, H-2).

f) Preparation of compound (8)

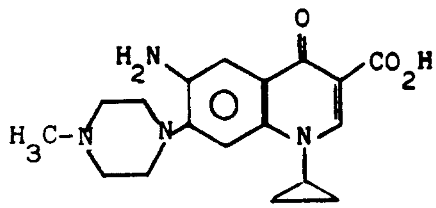

(8)

0.200 g of the aminoderivative (7) are suspended in 10 ml of an 1N NaOH solution and the suspension is refluxed for half an hour. The reaction mixture is cooled to room temperature and is then filtered. The filtrate is brought to pH 7 by addition of a 2N HCl solution and the precipitated white solid (70 mg) are collected by means of a (vacuum) pump; further 90 mg of the product are recovered from the filtrate by $CHCl_3$ extraction. Yield = 91%; m.p. = 255°C. The product consists of 6-amino-1-cyclopropyl-7-(4-methyl-1-piperazinyl)-1,4-dihydro-4-oxo-quinoline carboxylic acid (8).

$^1$H-NMR (DMSO-$d_6$): 1.10-1.40 (4H, m, cyclopropyl $CH_2$), 2.35 (3H, s, $NCH_3$), 2.45-2.80 and 2.95-3.20 (4H each, m, piperazine H), 3.65-4.00 (1H, m, cyclopropyl CH), 5.35 (2H, br s, $NH_2$), 7.50 (1H, s, H-8), 7.55 (1H, m, H-5), 8.50 (1H, s, H-2).

EXAMPLE 2

By working according to the operative conditions of example 1, starting from intermediate compound (5) and using piperazine as the required amine, is possible to obtain:
6-amino-1-cyclopropyl-7-(1-piperazinyl)-1,4-dihydro-4-oxo-quinoline carboxylic acid (9).;
m.p. > 300°C.
$^1$H-NMR (DMSO-$d_6$): 1.15-1.35 (4H, m, cyclopropyl $CH_2$), 2.75-3.35 (9H, m, piperazine H and NH), 3.70-3.95 (1H, m, cyclopropyl CH), 5.40 (1H, br s, $NH_2$), 7.50 (2H, s, H-5 and H-8), 8.45 (1H, s, H-2).

EXAMPLE 3

By working according to the operative conditions of example 1, starting from intermediate compound (5) and using pyrrolidine as the amine, it is possible to obtain:
6-amino-1-cyclopropyl-7-(1-pyrrolidinyl)-1,4-dihydro-4-oxo-quinoline carboxylic acid (10).;
m.p. = 275-276°C.
$^1$H-NMR (TFA): 1.20-1.80 (4H, m, cyclopropyl $CH_2$), 2.05-2.65 (4H, m, pyrrolidine H), 3.70-4.25 (5H, m, pyrrolidine H and cyclopropyl CH), 8.60 (1H, s, H-8), 9.25 (1H, s, H-5), 9.75 (1H, s, H-2).

EXAMPLE 4

By working according to the operative conditions of example 1, starting from intermediate compound (5) and using 2,6-dimethylpiperazine as the amine, it is possible to obtain:
6-amino-1-cyclopropyl-7-(3,5-dimethyl-1-piperazinyl)-1,4-dihydro-4-oxo-quinoline carboxylic acid (11);
m.p. > 300°C.
$^1$H-NMR (DMSO-$d_6$): 1.00-1.40 (10H, m, cyclopropyl $CH_2$ and piperazine $CH_3$), 3.10-3.55 (7H, m, piperazine H and NH), 3.60-3.80 (1H, m, cyclopropyl CH), 5.4 (2H, br s, $NH_2$), 7.50 (2H, s, H-5 and H-8), 8.45 (1H, s, H-2).

EXAMPLE 5

a) By working according to the operative conditions of example 1, starting from intermediate compound (5) and using 3-acetamido pyrrolidine as the amine, it is possible to obtain:

6-amino-1-cyclopropyl-7-(3-acetamido-1-pyrrolidinyl)-1,4-dihydro-4-oxo-quinoline carboxylic acid (12).
$^1$H-NMR (DMSO-$d_6$): 0.90-1.45 (4H, m, cyclopropyl $CH_2$), 1.85 (3H, s, $COCH_3$), 2.95-3.80 (7H, m, pyrrolidine H), 4.25-4.50 (1H, m, cyclopropyl CH), 5.30 (2H, br s, $NH_2$), 7.20 (1H, s, H-8), 7.40 (1H, s, H-5), 8.10 (1H, d, J = 7 Hz, NH), 8.45 (1H, s, H-2).

b) preparation of compound (13):

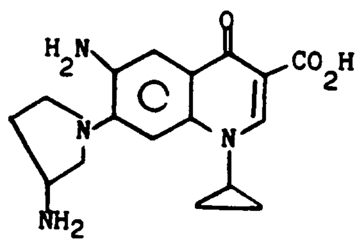

(13)

0.2 g of the acetylderivative (12) are suspended in 10 ml of a 1N NaOH solution and the whole is made to boil for 5 h. The reaction mixture is cooled to room temperature and then filtered. The filtrate is brought to pH 7 by adding a 2N HCl solution. The thus obtained solid is filtered and dried. Yield = 60%.
6-amino-1-cyclopropyl-7-(3-amino-1-pyrrolidinyl)-1,4-dihydro-4-oxo-quinoline carboxylic acid (13).
$^1$H-NMR (DMSO-$d_6$): 1.00-1.50 (4H, m, cyclopropyl $CH_2$), 3.20-4.10 (10H, m, pyrrolidine H and $NH_2$ and cyclopropyl CH), 5.70 (2H, br s, $NH_2$), 7.45 (1H, s, H-8). 7.50 (1H, s, H-5), 8.50 (1H,s, H-2).

EXAMPLE 6

By working according to the operative conditions of example 1, starting from intermediate compound (5) and using 2-methylpiperazine as the amine, it is possible to obtain:
6-amino-1-cyclopropyl-7-(3-methyl-1-piperazinyl)-1,4-dihydro-4-oxo-quinoline carboxylic acid (14).
$^1$H-NMR (DMSO-$d_6$): 0.85-1.40 (7H, m, cyclopropyl $CH_2$ and piperazine $CH_3$), 2.70-3.50 (8H, m, piperazine H and NH), 3.55-4.00 (1H, m, cyclopropyl CH), 5.35 ( (2H, br s, $NH_2$), 7.60 (2H, s, H-5 and H-8), 8.50 (1H, s, H-2).

EXAMPLE 7

By working according to the operative conditions described in example 1, starting from intermediate compound (5) and using thiomorpholine as the amine, it is possible to obtain the:
6-amino-1-cyclopropyl-7-(1-thiomorpholinyl)-1,4-dihydro-4-oxo quinoline carboxylic acid (15), having a m.p.>250 °C.
$^1$H-NMR (DMSO-$d_6$)$\delta$: 1.10-1.40 (4H, m, cyclopropylic $CH_2$), 2.80 and 3.20 (8H, m, thiomorpholinic H), 5.55 (2H, br s, $NH_2$), 7.60 (2H, s, H-5 and H-8), 8.60 (1H, s, H-2).

EXAMPLE 8

By working according to the operative conditions described in example 1, starting from intermediate compound (5) and using 4-hydroxypiperidine as the amine, it is possible to obtain the:
6-amino-1-cyclopropyl-7-(4-hydroxy-1-piperidinyl)-1,4-dihydro-4-oxo-quinoline carboxylic acid (16).
$^1$H-NMR (DMSO-$d_6$)$\delta$: 1.1-1.4 (4H, m, cyclopropylic $CH_2$), 1.60-3.9 (9H, m, piperidinic H), 5.7-6.7 (3H, br s, OH and $NH_2$), 7.7 (1H, s, H-8), 7.8 (1H, s, H-5), 8.6 (1H, s, H-2).

EXAMPLE 9

a) Preparation of compound (17):

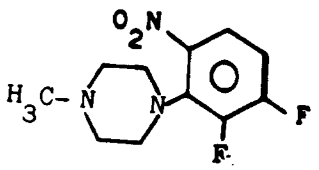

(17)

To a solution of 5 g (0.028 mol) of 2,3,4-trifluoronitrobenzene in 25 ml of toluene there are gradually added 2.82 g (0.028 mol) of N-methyl piperazine dissolved in 25 ml of toluene. The mixture is kept at room temperature under stirring for 12 h, then the reaction mixture is dried out under vacuum and the residue is chromatographed on silica gel, eluting with a 50-50 mixture of $CHCl_3$ and cyclohexane. There are thus obtained 3.8 g of 3,4-difluoro-2-(4-methyl-1-piperazinyl)-nitrobenzene (17) as an orange-red oil: Yield = 52%.
$^1$H-NMR ($CDCl_3$): 2.35 (3H, s, $CH_3$), 3.10-3.30 and 3.40 (4H each, m, piperazine H), 6.95 (1H, dt, J = 9 ad 7 Hz, H-5), 7.45 (1H, ddd, J = 9, 7 and 1.5 Hz, H-6).

b) Preparation of compound (18):

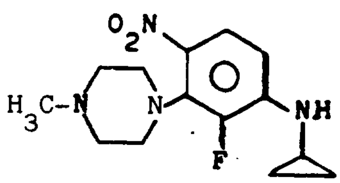

(18)

4 g (0.0155 mol) of 3,4-difluoro-2-(4-methyl-1-piperazinyl)-nitrobenzene are solubilized in 20 ml of HMPA (Hexamethylphosphoric triamide) and there are gradually added at room temperature 3.54 g (0.062 mol) of cyclopropylamine. The reaction mixture is first allowed to rest at 50 °C for 2 h, then poured into $H_2O$ and subjected to an extraction by means of ethyl acetate. The extracts are washed with $H_2O$, dried on $Na_2SO_4$ and dried out under vacuum. There are thus obtained 4.20 g of 4-cyclopropylamino-3-fluoro-2-(4-methyl-1-piperazinyl)-nitrobenzene (18) as a yellow semisolid. Yield = 92.52%.
$^1$H-NMR ($CDCl_3$): 0.40-0.95 (4H, m, cyclopropyl $CH_2$), 2.35 (3H, s, $CH_3$), 2.40-2.80 (5H, m, piperazine H and cyclopropyl CH), 3.05-3.35 (4H, m, piperazine H), 4.90 (1H, br s, NH), 6.75 (1H, t, J = 10.5 Hz, H-5), 7.65 (1H, dd, J = 10.5 ad 1.5 Hz, H-6).

c) Preparation of compound (19):

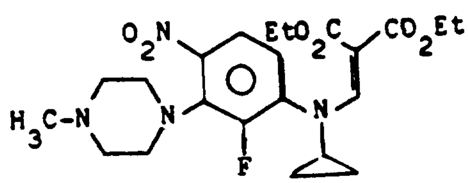

(19)

To 2.6 g (0.0088 mol) of compound (18) there are added 2.85 g (0.0132 mol) of diethylethoxymethylene malonate ad the mixture is made to react for 36 h at 140 °C. After such time, the mixture is cooled and purified by chromatographing on silica gel, eluting with a 50/50 mixture of cyclohexane and $CHCl_3$. There

are thus obtained 1.9 g of ethyl N-cyclopropyl-N-[2-fluoro-4-nitro-3-(4-methyl-1-piperaziniyl)-benzene]-aminomethylenmalonate (19) (yield = 50%) as a thick red oil.
$^1$H-NMR ($CDCl_3$): 0.45-0.95 (4H, m, cyclopropyl $CH_2$), 1.00-1.35 (6H, m, $CH_2CH_3$), 2.30 (3H, s, $CH_3$), 2.45-2.85 (5H, m, piperazine H and cyclopropyl CH), 3.00-3.35 (4H, m, piperazinic H), 3.90 and 4.20 (2H each, q, J = 7 Hz, $CH_2CH_3$), 6.80 (1H, dd, J = 9 and 7 Hz, H-6), 7.35 (1H, dd, J = 9 and 1.5 Hz, H-5), 7.50 (1H, s, vinyl H).

d) Preparation of compound (20):

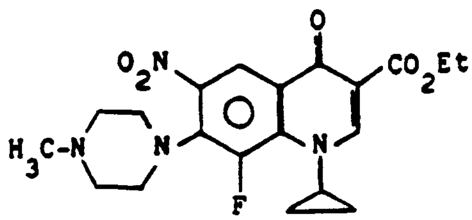

(20)

1.2 g (0.0027 mol) of compound (19) are made to react in an open pipe with 6 g of polyphosphoric acid. The reaction is continued for 2 h at 90 ° C and the reaction product is poured into a large amount of water and is then alkalized with $Na_2CO_3$. The thus formed precipitate is collected and washed more than once with $H_2O$. There are thus obtained 0.7 g of ethyl 1-cyclopropyl-8-fluoro-6-nitro-7-(4-methyl-1-piperazinyl)-1,4-dihydro-4-oxo-quinoline carboxylate (20). Yield = 62.05%; m.p. = 184-187 ° C.
$^1$H-NMR (DMSO-$d_6$): 1.00-1.45 (7H, m, cyclopropyl $CH_2CH_3$ and $CH_2$), 2.30 (3H, s, $CH_3$), 2.40-2.65 and 3.00-3.35 (4H each, m, piperazine H), 3.95-4.45 (3H, m, $CH_2CH_3$ and cyclopropyl CH), 8.30 (1H, s, H-5), 8.50 (1H, s, H-2).

e) Preparation of compound (21):

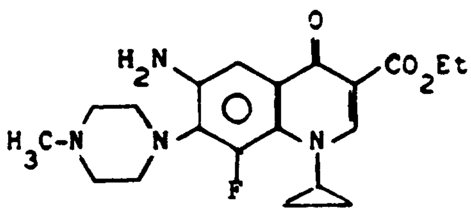

(21)

1.7 g (0.00407 mol) of compound (20) are solubilized in 25 ml of AcOH and there are added 1.65 g of iron powder (0.00285 mol). The reaction is continued for 1 h at 45 ° C, then iron is removed by filtration and the extract is alkalized and extracted with ethyl acetate. The organic phase is first washed with water, then dried on $Na_2SO_4$ and finally evaporated. The thus obtained residue is crystallized from cyclohexane/AcOEt. There are thus obtained 1.1 g (yield = 70%) of ethyl 6-amino-1-cyclopropyl-8-fluoro-7-(4-methyl-1-piperazinyl)-1,4-dihydro-4-oxo-quinoline carboxylate (21) as a light brown solid having m.p. 234-237 ° C.
$^1$H-NMR ($CDCl_3$): 0.95-1.55 (7H, m, cyclopropyl $CH_2CH_3$ and $CH_2$), 2.40 (3H, s, $CH_3$), 2.45-2.75 and 2.95-3.40 (4H each, m, piperazine H), 3.80-4.05 (1H, m, cyclopropyl CH), 4.30 (2H, q, J = 7 Hz, $CH_2CH_3$), 4.90 (2H, br s, $NH_2$), 7.75 (1H, s, H-5), 8.45 (1H, s, H-2).

f) Preparation of compound (22):

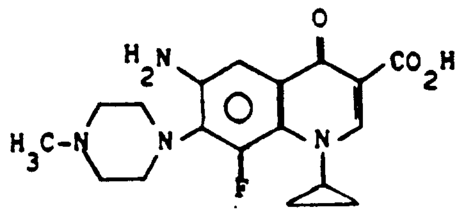

(22)

0.4 g (0.001 mol) of compound (21) are suspended in 10 ml of a 1N solution of NaOH and the suspension is refluxed. After 30', the reaction product is ice cooled and brought to pH 6-7 with diluted HCl. The thus formed precipitate is filtered ad washed more than once with $H_2O$. There are thus obtained 0.370 g of 6-amino-1-cyclopropyl-8-fluoro-7-(4-methyl-1-piperazinyl)-1,4-dihydro-4-oxo-quinoline carboxylic acid (22). Yield = 86.5%; m.p. > 300 ° C.
$^1$H-NMR (TFA): 1.35-1-75 (4H, m, cyclopropyl $CH_2$), 3.15 (3H, s, $CH_3$), 3.35-4.20 (8H, m, piperazine H), 4.35-4.80 (1H, m, cyclopropyl CH), 8.65 (1H, s, H-5), 9.45 (1H, s, H-2).

EXAMPLE 10

By working according to the operative conditions described in example 9, starting from 2,3,4-trifluoronitrobenzene and using thiomorpholine as the amine, it is possible to obtain the:
6-amino-1-cyclopropyl-8-fluoro-7-(1-thiomorpholinyl)-1,4-dihydro-4-oxo-quinoline carboxylic acid (23), having a m.p. > 300 ° C.
$^1$H-NMR (TFA)$\delta$:1.30-1.70 (4H, m, cyclopropylic $CH_2$), 3.20-3.75 (8H, m, thiomorpholinic H), 4.30-4.70 (1H, m, cyclopropylic CH), 8.60 (1H, s, H-5), 9.45 (1H, s, H-2).

EXAMPLE 11

a) Preparation of compound (24):

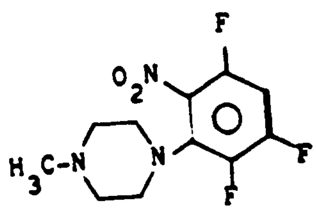

(24)

2 g (0.010 mol) of 2,3,4,6-trifluoronitrobenzene are solubilized in 20 ml of toluene and while keeping the solution ice cooled there are added drop by drop 0.513 g (0.010 mol) of N-methyl-piperazine dissolved in 10 ml toluene. Once the additions are over, the mixture is kept under stirring at room temperature, for 3 h, and is then dried out, thus obtaining a residue which, after cool washing with ethyl acetate, allows the separation of 0.660 g of 3,4,6-trifluoro-2-(4-methyl-1-piperazinyl)-nitrobenzene (24). Yield = 32.63%; m.p. = 100-104 ° C.
$^1$H-NMR ($CDCl_3$): 2.35 (3H, s, CH), 2.45-2.65 and 3.15-3.25 (4H each, m, piperazine H), 6.85 (1H, dt, J = 10.5 and 7 Hz, H-5). $^{19}$F-NMR ($CDCl_3$): 36.9 (1F, dd, J = 16 and 4 Hz, F-6), 33.6 (1F, dd, J = 20 and 4 Hz, F-4), 14.25 (1F, dd, J = 20 ad 12 Hz, F-3).

b) Preparation of compound (25):

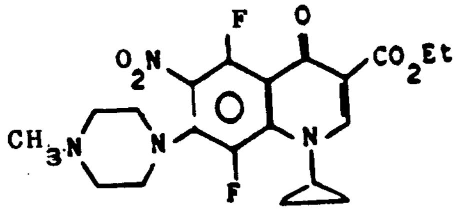

(25)

By working according to the operative conditions described in example 9 b), c) and d) and starting from intermediate compound (24), it is possible to obtain the ethyl 1-cyclopropyl-5,8-difluoro-6-nitro-7-(4-methyl-1-piperazinyl)-1,4-dihydro-4-oxo-quinoline carboxylate (25) having a m.p. = 206-208 °C. Crystallizes from AcOEt.

$^1$H-NMR ($CDCl_3$): 1.00-1.50 (7H, m, $CH_2CH_3$ and cyclopropyl $CH_2$), 2.35 (3H, s, $CH_3$), 2.40-2.65 and 3.05-3.40 (4H each, m, piperazine H), 3.80-4.00 (1H, m, cyclopropyl CH), 4.35 (2H, q, J = 6 Hz, $CH_2CH_3$), 8.45 (1H, s, H-2).

$^{19}$F-NMR ($CDCl_3$): 19.10 (1F, d, J = 16 Hz, F-5), 16.20 (1F, d, J = 16 Hz, F-8).

c) Preparation of compound (26):

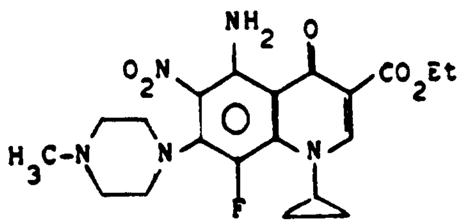

(26)

To the solution of 1 g (0.0023 mol) of compound (25) in 20 ml of EtOAc there are added 3 ml of $NH_4OH$ (30% solution) and the mixture is placed into an autoclave at 80 °C for 50'. The reaction mixture is then poured in $H_2O$ and extracted more than once with ethyl acetate. The extract is washed with $H_2O$, dried on $Na_2SO_4$ and dried out in an apparatus commonly known as ROTAVAPOR. There is obtained a solid which, after crystallization from ethyl acetate, amounts to 0.88 g. Yield = 95.0%; m.p. = 102-104 °C. Said solid consists of ethyl 5-amino-1-cyclopropyl-8-fluoro-6-nitro-7-(4-methyl-1-piperazinyl)-1,4-dihydro-4-oxo-quinoline carboxylate (26).

$^1$H-NMR ($CDCl_3$): 0.90-1.55 (7H, m, $CH_2CH_3$ and cyclopropyl $CH_2$), 2.45 (3H, s, $CH_3$), 2.50-2.75 and 3.25-3.45 (4H each, m, piperazine H), 3.85-4.10 (1H, m, cyclopropyl CH), 4.50 (2H, q, J = 6 Hz, $CH_2CH_3$), 8.40 (1H, s, H-2), 9.10 (2H, br s, $NH_2$).

$^{19}$F-NMR ($CDCl_3$): 16.00 (1F, s).

d) Preparation of compound (27):

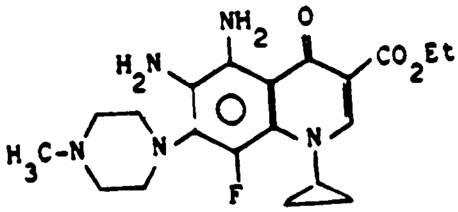

(27)

0.350 g (0.00081 mol) of compound (26) are solubilized in 150 ml of anhydrous EtOH, 0.07 g of Raney nickel are added, the reduction mixture is ice cooled and a stream of $H_2$ is allowed to pass through. The ice cooling is maintained for 30' and the mixture is kept for further 30' at room temperature, then is filtered on celite and the filtrate is dried out (in ROTAVAPOR). The residue, cool treated with $CCl_4$, allows the precipitation of 0.170 g (yield = 52.30%) of ethyl 5,6-diamino-1-cyclopropyl-8-fluoro-7-(4-methyl-1-piperazinyl)-1,4-dihydro-4-oxo-quinoline carboxylate (27) in the form of a yellow solid.

$^1$H-NMR ($CDCl_3$): 0.70-1.25 (4H, m, cyclopropyl $CH_2$), 1.40 ( 3H, t, J = 6 Hz, $CH_2CH_3$), 2.35 (3H, s, $CH_3$), 2.40-3.60 (8H, m, piperazine H), 3.70-4.20 (3H, m, cyclopropyl CH and $NH_2$ in C-6), 4.40 (2H, q, J = 6 Hz, $CH_2CH_3$), 6.35 (2H, br s, $NH_2$ in C-5), 8.35 (1H, s, H-2).

e) Preparation of compound (28):

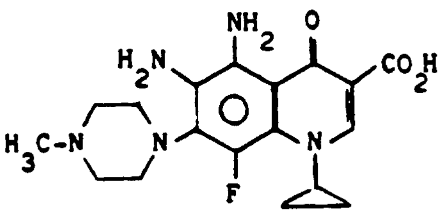

(28)

0.150 g of compound (27) are suspended in 4 ml of a 1N solution of NaOH and refluxed for 30'. The solution is then ice cooled and treated with diluted HCl until forming a light yellow precipitate (28) (pH 8). The precipitate is washed with $H_2O$ and then crystallized from MeOH. Yield = 64.5%; m.p. = 281-283 °C.

5,6-diamino-1-cyclopropyl-8-fluoro-7-(4-methyl-1-piperazinyl)-1,4-dihydro-4-oxo-quinoline carboxylic acid (28).

$^1$H-NMR (DMSO-$d_6$): 1.00-1.30 (4H, m, cyclopropyl $CH_2$), 2.35 (3H, s, $CH_3$), 2.60-3.90 (8H, m, piperazine H), 3.95-4.15 (1H, m, cyclopropyl CH), 5.05 (2H, br s, $NH_2$ in C-6), 6.95 (2H, br s, $NH_2$ in C-5), 8.35 (1H, s, H-2).

EXAMPLE 12

a) Preparation of compound (29):

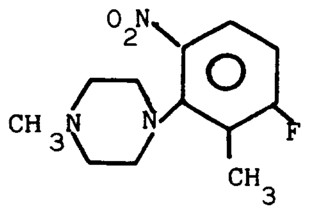

(29)

2 g (0.0110 mol) of 2,6-difluoro-3-nitrotoluene are solubilized in 20 ml of toluene and to this solution there are added 1.15 g (0.0115 mol) of N-methylpiperazine and 2.33 g (0.023 mol) of triethylamine. The mixture is placed in an autoclave for 20 h at 125 ° C. The solvent is then evaporated ad the residual oil is chromatographed on a silica gel column, by using $CH_2Cl_2$ as the eluting agent. There are thus obtained 700 mg of 6-fluoro-3-nitro-2-(4-methyl-1-piperazinyl)-toluene (29) in the form of an oil.
$^1$H-NMR ($CDCl_3$)$\delta$: 2.3 (3H, d, $CH_3$), 2.35 (3H, s, N-$CH_3$), 2.4-2.7 and 2.99-3.2 (4H each, m, piperazinic H), 6.85 (1H, t, J = 9 Hz, H-5), 7.45 (1H, dd, J = 9 Hz, H-4).

b) Preparation of compound (30):

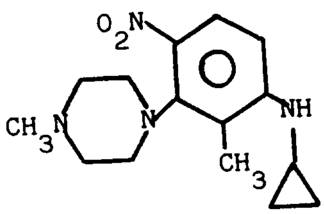

(30)

0.9 g (0.0035 mol) of compound (29) are dissolved in 15 ml of HMPA and to this solution there are added 0.825 g (0.014 mol) of cyclopropylamine. The reaction mixture is maintained at 60 ° C for 20 h, then poured into $H_2O$ and subjected to extraction more than once with EtOAc. The organic extracts are repeatedly washed with $H_2O$ and dehydrated on $Na_2SO_4$. The residue obtained from the evaporation of the solvent is chromatographed on a column of silica gel, by eluting with a mixture $CH_2Cl_2$:EtOAc:MeOH (97:2:1). There are thus obtained 0.4 g of 6-cyclopropylamino-2-(4-methyl-1-piperazinyl)-3-nitrotoluene(30) in the form of a oil.
$^1$H-NMR ($CDCl_3$) $\delta$: 0.50-0.95 (4H, m, cyclopropylic $CH_2$), 2.05 (3H, s, $CH_3$), 2.35 (3H, s, N-$CH_3$), 2.4-2.6 ( 5H, m, piperazinic H and cyclopropylic H), 2.9-3.2 (4H, m, piperazinic H), 6.8 (1H, d, J = 9 Hz, H-5), 7.6 (1H, d, J = 9 Hz, H-4).

c) preparation of compound (31):

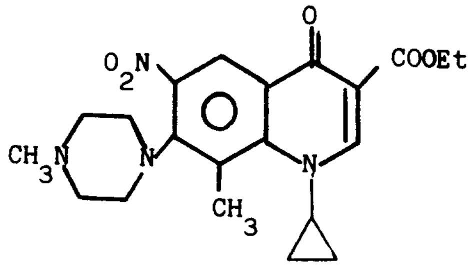

(31)

To 0.500 g (0.0017 mol) of compound (30) there are added 0.6 g of EMME (0.0027 mol) and the mixture is maintained at 140° C for 36 h, then is cooled down to 80° C and there are added 2.5 g of polyphosphoric acid. The reaction is allowed to continue for 1 h, then the reaction mixture is poured into $H_2O$ and ice and is alkalized up to pH 8 by means of NaOH. There is thus formed a precipitate which is filtered. There are thus obtained 0.240 g of ethyl 1-cyclopropyl-8-methyl-6-nitro-7-(4-methyl-1-piperazinyl)-1,4-dihydro-4-oxo-quinoline carboxylate (31) in the form of a yellow solid.
$^1$H-NMR ($CDCl_3$) δ: 0.75-1.5 (7H, m, $CH_2CH_3$ and cyclopropylic $CH_2$), 2.4 (3H, s, N-$CH_3$), 2.4-2.6 (5H, m, piperazinic H and cycloproprylic H), 2.7 (3H, s, $CH_3$), 2.95-3.25 (4H, s, piperazinic H), 4.4 (2H, q, J=7 Hz, $CH_2CH_3$), 8.5 (1H, s, H-5), 8.6 (1H, s, H-2).
Melting Point: 210-213° C.

d) Preparation of compound (32):

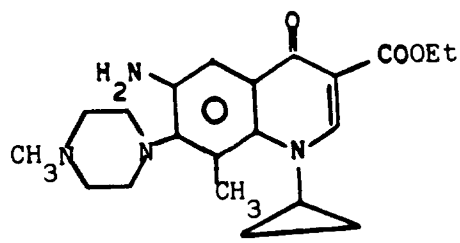

(32)

0.240 g (0.57 mol) of compound (31) are dissolved in 5 ml of 2-methoxy-ethanol. To this solution there are added 0.1 g of Raney nickel and a stream of $H_2$ is allowed to pass through for 1 h. The reaction mixture is then filtered on celite and the solution is evaporated under vacuum. The solid residue is washed with little EtOH and there are thus obtained 0.240 g of ethyl 6-amino-1-cyclopropyl-8-methyl-7-(4-methyl-1-piperazinyl)-1,4-dihydro-4-oxo-quinoline carboxylate (32).
$^1$H-NMR ($CDCl_3$) δ:0.8-1.55 (7H, m, $CH_2CH_3$ and cyclopropylic $CH_2$), 2.4 (3H, s, N-$CH_3$), 2.6 (3H, s, $CH_3$), 2.8-3.6 (8H, m, piperazinic H), 3.75-4.00 (1H, m, cyclopropylic H), 4.20 (2H, br s, $NH_2$), 4.40 (2H, q, J=7 Hz, $CH_2CH_3$), 7.60 (1H, s, H-5), 8.60 (1H, s, H-2).
Melting Point = 223-225° C.

e) Preparation of compound (33):

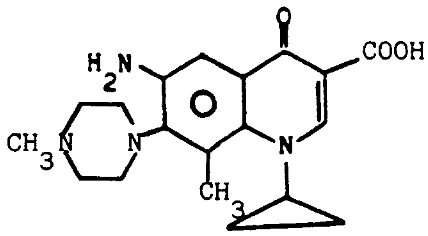

(33)

There is prepared a 50/50 mixture of 2.5 ml of EtOH and 2.5 ml of HCl and there are added 0.2 g (0.52 mmol) of compound (32). The solution is refluxed for 6 h and after cooling there occurs the precipitation of a white solid which is filtered. There are thus obtained 0.070 g of 6-amino-1-cyclopropyl-8-methyl-7-(4-methyl-1-piperazinyl)-1,4-dihydro-4-oxo-quinoline carboxylic acid chlorohydrate (33).
$^1$H-NMR (DMSO-$d_6$) δ: 0.7-1.3 (4H, m, cyclopropylic $CH_2$), 2.6 (3H, s, $CH_3$, 2.8 (3H, d, $NHCH_3$), 3.2-3.7 (9H, m, piperazinic H and cyclopropylic H), 4.3 (2H, br s, $NH_2$), 7.5 (1H, s, H-5), 8.65 (1H, s, H-2). Melting Point > 300 ° C.

EXAMPLE 13

By working according to the operative conditions described by example 12, starting from 2,6-difluoro-3-nitrotoluene and using piperazine as the amine it is possible to obtain the 6-amino-1-cyclopropyl-8-methyl-7-(1-piperazinyl)-1,4-dihydro-4-oxo-quinoline carboxylic acid chlorohydrate (34).
$^1$H-NMR (DMSO-$d_6$) δ:0.8-1.4 (4H, m, cyclopropylic $CH_2$), 2.65 (3H, s, $CH_3$), 2.95-3.70 (10H, m, piperazinic H, NH and cyclopropylic H), 4.5 (2H, br s, $NH_2$), 7.4 (1H, s, H-5), 8.55 (1H, s, H-2).

EXAMPLE 14

By working according to the operative conditions described by example 12, starting from 2,6-difluoro-3-nitrotoluene and using thiomorpholine as the amine, it is possible to obtain the 6-amino-1-cyclopropyl-8-methyl-7-(1-thiomorpholinyl)-1,4-dihydro-4-oxo-quinoline carboxylic acid (35).
$^1$H-NMR (DMSO-$d_6$) δ: 0.8-1.4 (4H, m, cyclopropylic $CH_2$), 2.65 (3H, s, $CH_3$), 2.8-3.4 (8H, m, thiomorpholinic H), 3.7-3.9 (1H, m, cyclopropylic CH), 4.8 (2H, br s, $NH_2$), 7.6 (1H, s, H-5), 8.60 (1H, s, H-2).

EXAMPLE 15

By working according to the operative conditions described by example 12, starting from 2,6-difluoro-3-nitrotoluene and using 1,2,3,4-tetrahydroisoquinoline as the amine, it is possible to obtain the 6-amino-1-cyclopropyl-8-methyl-7-[2-(1,2,3,4-tetrahydroisoquinolinyl)]-carboxylic acid (36).
$^1$H-NMR ($CDCl_3$) δ: 0.8-1.4 (4H, m, cyclopropylic $CH_2$), 2.6 (3H, s, $CH_3$). 2.85-4.4 (6H, m, isoquinolinic $CH_2$), 7.1-7.3 (4H, m, isoquinoline aromatics), 7.5 (1H, s, H-5), 8.7 (1H, s, H-2).

EXAMPLE 16

a) Preparation of compound (37):

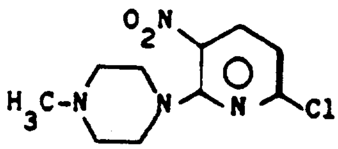

(37)

2 g (0.0103 mol) of 2,6-dichloro-3-nitropyridine are solubilized in 15 ml of toluene and to the solution there are slowly added 1.04 g (0.00103 mol) of N-methyl piperazine, solubilized in toluene (10 ml). Stirring is continued for 5 h at room temperature then the formed precipitate is collected and washed with Me OH. There are thus obtained 2.42 g (yield = 92%) of 6-chloro-3-nitro-2-(4-methyl-1-piperazinyl)-pyridine (37) having m.p. = 241-243 ° C.

$^1$H-NMR(DMSO-$d_6$): 2.80 (3H, s, $CH_3$), 3.00-3.40 and 3.45-3.85 (4H each, m, piperazine H), 7.05 (1H, d, J = 9 Hz, H-5), 8.35 (1H, d, J = 9 Hz, H-4).

b) Preparation of compound (38):

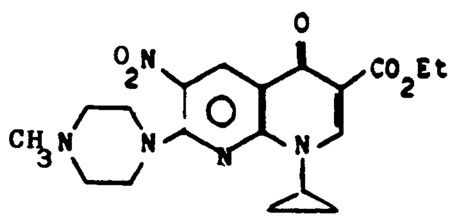

(38)

By working according to the operative conditions described in examples 9 b) c) d) starting from the intermediate (37) it is possible to obtained:

Ethyl 1-cyclopropyl-6-nitro-7-(4-methyl-1-piperazinyl)-1,4-dihydro-oxo-1,8-naphthiridine carboxylate (38). Crystallizes from ethyl acetate; m.p. = 187-188 ° C.

$^1$H-NMR ($CDCl_3$): 1.00-1.55 (7H, m, $CH_2CH_3$ and cyclopropyl $CH_2$), 2.35 (3H, s, $CH_3$), 2.50-2.70 (4H, m, piperazine H), 3.40-3.80 (5H, m, piperazine H and cyclopropyl CH), 4.40 (2H, q, J = Hz, $CH_2CH_3$), 8.50 (1H, s, H-5), 8.95 (1H, s, H-2).

c) Preparation of compound (39):

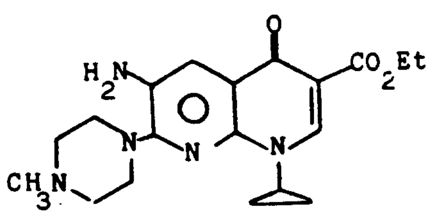

(39)

0.5 g (0.0012 mol) of compound (38) are solubilized in 70 ml of EtOH and after having added 0.1 g of Raney nickel a stream of $H_2$ is allowed to pass through for 1 h. The reaction product is filtered on celite,

dried out under vacuum and allows, by addition of $CCl_4$ and cyclohexane, the precipitation of 0.350 g (yield = 78.65%) of a product consisting of ethyl 6-amino-1-cyclopropyl-7-(4-methyl-1-piperazinyl)-1,4-dihydro-4-oxo-1,8-naphthiridine carboxylate (39).
The product crystallized from ethyl acetate is showing a m.p. of 231-232 ° C.
$^1$H-NMR ($CDCl_3$): 0.90-1.30 (4H, m, cyclopropyl $CH_2$), 1.40 (3H, t, J = 7 Hz, $CH_2CH_3$), 2.40 (3H, s, $CH_3$), 2.55-2.70 (4H, m, piperazine H), 3.35-3.65 (5H, m, piperazine H and cyclopropyl CH), 3.95 (2H, br s, $NH_2$), 4.40 (2H, q, J = 7 Hz, $CH_2CH_3$), 7.90 (1H, s, H-5), 8.45 (1H, s, H-2).

d) Preparation of compound (40):

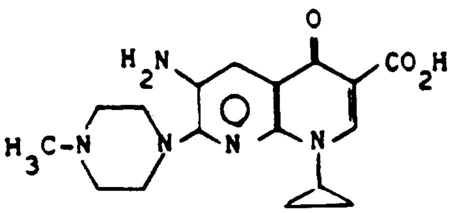

(40)

0.2 g (0.0054 mol) of compound (39) are solubilized in 2.5 ml of EtOH and 2.5 ml of a 6N solution of HCl and then refluxed for 12 h. By cooling there occurs the precipitation of a yellow solid which is then collected by means of a (vacuum) pump (0.120 g). After crystallization from EtOH/$H_2O$, the melting point is > 300 ° C. The product consists of the chlorhydrate of the 6-amino-1-cyclopropyl-7-(4-methyl-1-piperazinyl)-1,4-dihydro-4-oxo-1,8-naphthyridine carboxylic acid (40).
$^1$H-NMR (DMSO-$d_6$): 1.05-1.32 (4H, m, cyclopropyl $CH_2$), 2.85 (3H, d, J = 4.5 Hz, $CH_3$), 3.20-4.35 (9H, m, piperazine H and cyclopropyl CH), 6.30-6.60 (3H, m, $NH_2$ and $NHCH_3$), 7.70 (1H, s, H-5), 8.55 (1H, s, H-2).

EXAMPLE 17

a) Preparation of compound (41):

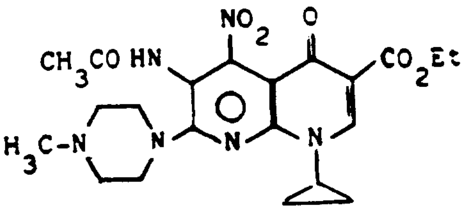

(41)

0.3 g (0.00080 mol) of compound (39) are made to react with 3.6 ml of AcOH and 1.2 ml of $Ac_2O$ at room temperature for 1 h. The mixture is then brought to -5 ° C and there are added further 1.05 ml of $Ac_2O$ and then, drop by drop, the cooled nitrating mixture consisting of 0.375 ml of fuming $HNO_3$ and 0.75 ml of $Ac_2O$. The reaction is continued for 1 h at -5 ° C, then the reaction product is poured into $H_2O$ and ice, alkalized and subjected more than once to extraction with $CH_2Cl_2$. The extract is washed with water and dried in ROTAVAPOR. The purification is carried out by chromatographing on silica gel and eluting with increasing concentrations of MeOH in $CHCl_3$ up to 7%. There are thus obtained 0.265 g of ethyl 6-acetamido-1-cyclopropyl-5-nitro-7-(4-methyl-1-piperazinyl)-1,4-dihydro-4-oxo-1,8-naphthiridine carboxylate (41).
$^1$H-NMR ($CDCl_3$): 1.00-1.50 (7H, m, cyclopropyl $CH_2CH_3$ and $CH_2$), 2.20 (3H, s, $CH_3$), 2.40 (3H, s, $COCH_3$), 2.45-2.70 (4H, m, piperazine H), 3.35-3.80 (1H, m, cyclopropyl CH), 3.80-4.05 (4H, m, piperazine H), 4.25 (2H, q, J = 7 Hz, $CH_2CH_3$), 8.40 (1H, s, H-2), 8.80 (1H, br s, NH).

b) Preparation of compound (42):

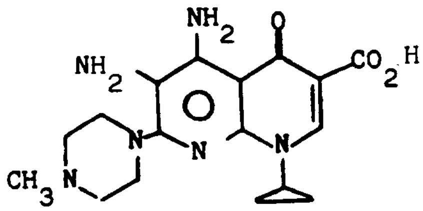

(42)

By working as in example 11 d),e), it is possible to obtain the 5,6-diamino-1-cyclopropyl-7-(4-methyl-1-piperazinyl)-1,4-dihydro-4-oxo-1,8-naphthyridine carboxylic acid (42).
$^{1}$H-NMR (DMSO-$d_6$) $\delta$: 1.0-1.4 (4H, m, cyclopropylic $CH_2$), 2.25 (3H, d, N-$CH_3$), 2.8-3.3 (8H, m, piperazinic $CH_2$), 3.7-3.9 (1H, m, cyclopropylic CH), 8.5 (1H, s, H-2).

EXAMPLE 18

a) Preparation of compound (43):

(43)

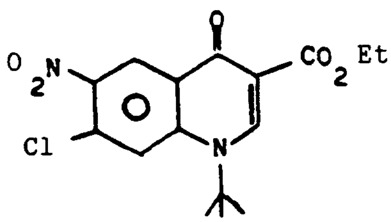

By working according to the operative conditions described by example 1 b), c), starting from intermediate compound (3) and using t-butylamine as the amine, instead of cyclopropylamine, it is possible to obtain the ethyl 1-terbutyl-7-chloro-6-nitro-1,4-dihydro-4-oxo-quinoline carboxylate (43).

| Analysis (%) for $C_{16}H_{17}ClN_2O_5$: | | | | |
|---|---|---|---|---|
| Calculated | C = 54.47 | H = 4.86 | N = 7.94 | Cl = 10.05 |
| Found: | C = 54.28 | H = 4.78 | N = 7.85 | Cl = 9.80 |

b) Preparation of compound (44):

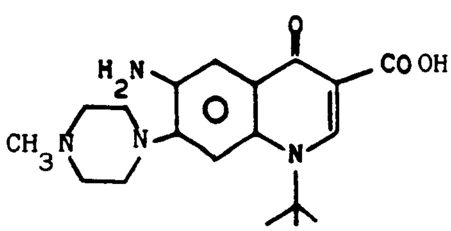

(44)

By working according to the operative conditions described in example 1 d), e), f), starting from intermediate (43) and using N-methyl-piperazine as the amine, there is obtained the 6-amino-1-terbutyl-7-(4-methyl-1-piperazinyl)-1,4-dihidro-4-oxo-quinoline carboxylic acid (44).
$^1$H-NMR ($CDCl_3$) $\delta$:1.9 (9H, s, t butyl), 2.45 (3H, s, N-$CH_3$), 2.6-3.25 (8H, m, piperazinic $CH_2$), 4.3 (2H, br s, $NH_2$), 7.5 (1H, s, H-8), 7.75 (1H, s, H-5), 8.95 (1H, s, H-2).

EXAMPLE 19

By working according to the operative conditions described in example 1 d), e), f), starting from intermediate compound (43) and using piperazine as the amine, there is obtained the 6-amino-1-terbutyl-7-(1-piperazinyl)-1,4-dihydro-4-oxo-quinoline carboxylic acid (45).
$^1$H-NMR ($CDCl_3$) $\delta$: 1.85 (9H, s, terbutyl), 2.65-3.20 (9H, m, piperazinic H and NH), 4.2 (2H, br s, $NH_2$), 7.50 (1H, s, H-8), 7.75 (1H, s, H-5), 8.95 (1H, s, H-2).

EXAMPLE 20

By working according to the operative conditions described in example 1 d) e ) f), starting from intermediate compound (43) and using thiomorpholine as the amine, there is obtained the 6-amino-1-terbutyl-7-(1-thiomorpholinyl)-1,4-dihydro-4-oxo-quinoline carboxylic acid (46).
$^1$H-NMR (DMSO-$d_6$) $\delta$: 1.8 (9H, s, t-but.), 2.80-3.40 (8H, m, thiomorpholinic H), 5.6 (2H, br s, $NH_2$), 7.70 (1H, s, H-5), 7.85 (1H, s, H-8).8.9 (1H, s, H-2).

EXAMPLE 21

By working according to the operative conditions described in example 1 d) e) f), starting from intermediate compound (43) and using 1,2,3,4-tetrahydroisoquinoline as the amine, there is obtained the 6-amino-1-terbutyl-7-[2-(1,2,3,4-tetrahydroisoquinolinyl)]-1,4-dihydro-4-oxo-quinoline carboxylic acid (47).
$^1$H-NMR ($CDCl_3$) $\delta$: 1.7 (9H, s, terbutyl), 2.85-4.40 (6H, m, isoquinolinic $CH_2$) 7.5 (1H, s, H-8), 7.8 (1H, s, H-5), 8.95 (1H, s, H-2).

EXAMPLE 22

By working according to the operative conditions described in example 1 d) e) f), starting from intermediate compound (43) and using 1-(2-pyridyl)-piperazine as the amine, there is obtained the 6-amino-1-terbutyl-7-[4-(2-pyridyl)-1-piperazinyl]-1,4-dihydro-4-oxo-quinoline carboxylic acid (48).
$^1$H-NMR ($CDCl_3$) $\delta$: 1.9 (9H, s, t-butyl), 3.15-3.85 (8H, m, piperazinic $CH_2$), 4.3 (2H, br s, $NH_2$), 6.65-6.8 (2H, m, H-8 and pyridinic H-5), 7.5-7.6 (2H, m, H-8 and pyridinic H-3), 7.8 (1H, s, H-5), 8.25 (1H, dd, pyridinic H-6), 8.95 (1H, s, H-2).

EXAMPLE 23

a) Preparation of compound (49):

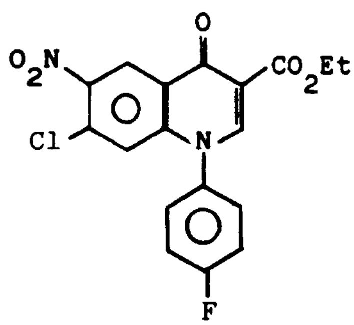

(49)

By working according to the operative conditions described in example 1 b) c), starting from intermediate (3) and using p-fluoroaniline instead of cyclopropylamine, it is possible to obtain the ethyl 1-(4-fluorophenyl)-7-chloro-6-nitro-1,4-dihydro-4-oxo-quinoline carboxylate (49).

| Analysis (%) for $C_{24}H_{12}ClFN_2O_5$: | | | | |
|---|---|---|---|---|
| Calculated | C 62.28 | H 2.61 | N 6.05 | Cl 7.66 |
| Found | C 61.95 | H 2.55 | N 6.00 | Cl 7.46 |

b) Preparation of compound (50):

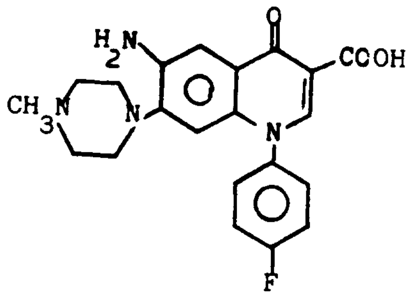

(50)

By working according to the operative conditions described in example 12 c) d) e), starting from the intermediate compound (49) and using N-methyl-piperazine as the amine, there is obtained the 6-amino-1-(4-fluorophenyl)-7-(4-methyl-1-piperazinyl)-1,4-dihydro-4-oxo-quinoline carboxylic acid (50).
$^1$H-NMR (DMSO-$d_6$) $\delta$: 2.75 (3H, s, N-$CH_3$), 2.8-3.35 (8H, m, piperazinic $CH_2$), 6.45 (1H, s, H-8), 7.4-7.7 (5H, m, aromatic H and H-5), 8.5 (1H, s, H-2).

EXAMPLE 24

By working according to the operative conditions described in example 12 c) d) e), starting from the intermediate compound (49) and using thiomorpholine as the amine, there is obtained the 6-amino-1-(4-fluorophenyl)-7-(1-thiomorpholinyl)-1,4-dihydro-4-oxo-quinoline carboxylic acid (51).
$^1$H-NMR (DMSO-$d_6$) $\delta$: 2.75-3.40 (8H, m, thiomorpholinic H), 6.40 (1H, s, H-8), 7.4-7.7 (5H, m, aromatic H and H-5), 8.5 (1H, s, H-2).

EXAMPLE 25

By working according to the operative conditions described in example 12 c) d) e), starting from intermediate compound (49) and using 1,2,3,4-tetrahydroisoquinoline as the amine there is obtained the 6-amino-1-(4-fluorophenyl)-7-[2-(1,2,3,4-tetrahydroisoquinolinyl)]-1,4-dihydro-4-oxo-quinoline carboxylic acid (52).
$^1$H-NMR (DMSO-$d_6$) $\delta$: 3.6-4.6 (6H, m, isoquinolinic $CH_2$), 6.50 (1H, s, H-8), 7.2-7.7 (9H, m, aromatic H and H-5), 8.4 ( 1H, s, H-2).

SUMMARIZING TABLE EXEMPLIFIED COMPOUNDS

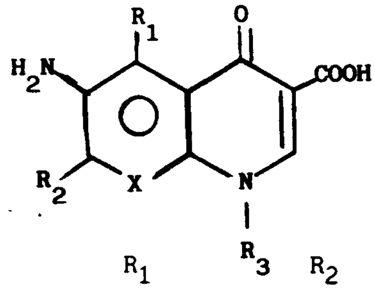

| COMPOUND | X | $R_1$ | $R_2$ | $R_3$ |
|---|---|---|---|---|
| 8 | CH | H | 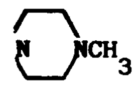 | 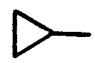 |
| 9 | CH | H | 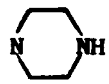 | 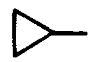 |
| 10 | CH | H | 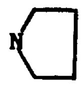 | 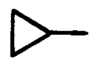 |
| 11 | CH | H | 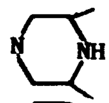 | 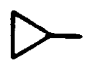 |
| 12 | CH | H | 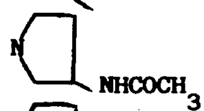 | 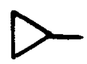 |
| 13 | CH | H | 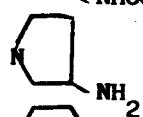 | 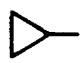 |
| 14 | CH | H | 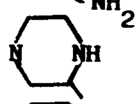 | 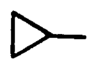 |
| 15 | CH | H | 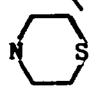 | 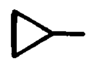 |
| 16 | CH | H | 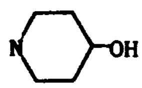 | 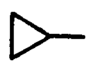 |
| 22 | CF | H | 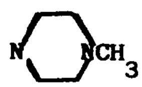 | 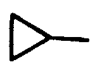 |
| 23 | CF | H | 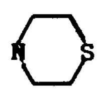 | 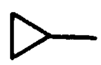 |
| 28 | CF | $NH_2$ | 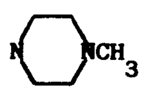 | 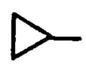 |
| 33 | $CCH_3$ | H | 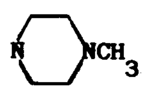 | 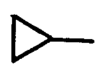 |

| COMPOUND | X | $R_1$ | $R_2$ | $R_3$ |
|---|---|---|---|---|
| 34 | $CCH_3$ | H | 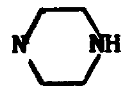 | 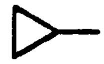 |
| 35 | $CCH_3$ | H | 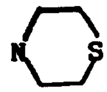 | 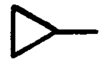 |
| 36 | $CCH_3$ | H | 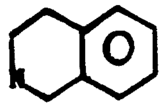 | 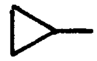 |
| 40 | N | H | 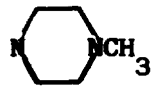 | 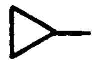 |
| 42 | N | $NH_2$ | 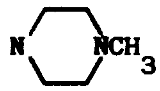 | 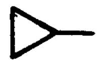 |
| 44 | CH | H | 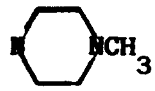 | 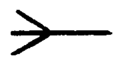 |
| 45 | CH | H | 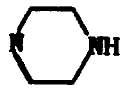 | 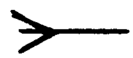 |
| 46 | CH | H | 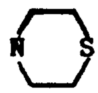 | 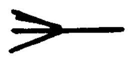 |
| 47 | CH | H | 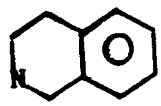 | 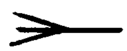 |
| 48 | CH | H | 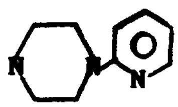 | 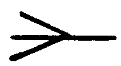 |
| 50 | CH | H | 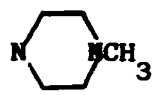 | 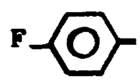 |
| 51 | CH | H | 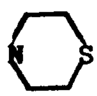 | 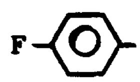 |
| 52 | CH | H | 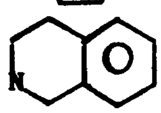 | 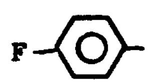 |

Pharmacological tests:

The compounds prepared according to the examples hereinabove were employed for the tests for evaluating their pharmacological characteristics.

As to the antibacterial activity, there was determined the Least (Minimum) Inhibiting Concentration (MIC), by working according to the following operative conditions.

Microorganisms

There were used suspensions of test germs (5 gram-positive and 15 gram-negative) having a known titre, kept freezed at -80 ° C and diluted, at the time of use, in peptonized water up to a concentration of 1-2.$10^7$ cfu/ml.

Products

A few mg of the products, according to the invention were weighed sufficient for preparing parent solutions in DMSO + NaOH (1N) containing 2560 µg/ml (64µg/ml/dish). From the thus prepared parent solutions there were prepared 12 serial dilutions (1:2) down to the concentration of 1.25 µg/ml.

Preparation of the dishes

0.5 ml of the different dilutions of each product were distributed on dishes containing 19.5 ml of agarized medium (TSA), maintained in a fluid state at the temperature of 50 ° C. After slight stirring, the dishes were made to solidify and dry under hood (laminar flow).

Performance of the MIC tests

The MIC tests (Least or "Minimum" Inhibiting Concentration) were performed by using a Steers multiwell inoculator having 36 wells, each containing 200 µl of bacterial suspension.

After inoculation, the dishes were placed into a thermostat and checked after 24 h of incubation at 35 ° C.

The MIC is defined as the lowermost product concentration (µg/ml) inhibiting the visible growth of the microorganisms.

The results are recorded on Table 1, whereon it is also recorded, as a blank, the MIC of RUFLUXACIN, a known drug showing a high antibacterial activity.

In addition to the high antibacterial activity, as it can be inferred from Table 1, the compounds according to the invention have also the following important features: they show a greater solubility in water, with respect to the quinolones of the prior art, and give rise to reduced side effects, at the level of the central nervous system, as they pass with greater difficulty through the blood-brain barrier.

Because of their features, the 6-aminoquinolones of the present invention can be employed in the preparation of pharmaceutical compositions containing an effective dosage of the same compounds, in admixture with pharmacologically acceptable excipients and diluting agents.

Said compositions can be therapeutically employed in the treatment of affections bound to bacterial aetiology.

TABLE N. 1 - Minimum Inhibiting Concentration (µg/ml)

| MICROORGANISMS COMPOUND: | RUFLOXACIN | 8 | 9 | 10(*) | 11(*) | 14 | 15 | 16 | 22 |
|---|---|---|---|---|---|---|---|---|---|
| Gram + | | | | | | | | | |
| Staphylococcus aureus MPR 5 | 1 | 2 | 64 | 128 | 8 | 16 | 0.5 | 4 | 4 |
| Staphylococcus aureus ATCC 6538 | 0.5 | 2 | 64 | 128 | 8 | 16 | 0.5 | 4 | 4 |
| Staphylococcus epidermidis HCF Berset C. | 2 | 4 | 16 | 128 | 4 | 4 | 2 | 4 | 8 |
| Staphylococcus epidermidis CPHL A2 | 1 | 2 | 16 | 128 | 2 | 4 | 1 | 2 | 4 |
| Streptococcus facealis LEP Br. | 8 | 32 | 128 | 128 | 32 | 32 | 8 | 8 | 64 |
| Log | 0.181 | 0.602 | 1.625 | 2.107 | 0.843 | 1.023 | 0.120 | 0.602 | 0.903 |
| Geometrical Average | 1.515 | 4.00 | 42.224 | 128.0 | 6.964 | 10.556 | 1.320 | 4.00 | 8.00 |
| Gram - | | | | | | | | | |
| Escherichia coli CNUR 1 | 0.5 | 0.25 | 0.25 | 16 | 0.25 | 0.12 | 0.5 | 2 | 0.12 |
| Escherichia coli CNUR 8 | 0.5 | 0.12 | 0.25 | 8 | 0.25 | 0.12 | 0.12 | 1 | 0.12 |
| Escherichia coli 120 | 0.25 | 0.12 | 0.25 | 4 | 0.12 | 0.06 | 0.12 | 0.5 | 0.06 |
| Escherichia coli ATCC 8739 | 0.5 | 0.25 | 0.25 | 0.25 | 0.03 | 0.03 | 0.03 | 0.03 | 1.00 |
| Escherichia coli ISF 432 | 0.06 | 0.03 | 0.03 | 0.25 | 0.03 | 0.03 | 0.03 | 0.03 | 0.06 |
| Enterobacter cloacae OMNF 1153 | 1 | 0.25 | 0.5 | 16 | 0.5 | 0.5 | 0.5 | 2 | 0.25 |
| Enterobacter cloacae OMNF 1174 | 0.5 | 0.25 | 0.25 | 8 | 0.5 | 0.25 | 0.5 | 2 | 0.25 |
| Acinebacter calcoloaceticus OSMPV 1130 | 2 | 4 | 16 | 128 | 8 | 16 | 1 | 2 | 8 |
| Proteus vulgaris CNUR 6 | 16 | 64 | 32 | 128 | 64 | 32 | 128 | 128 | 32 |
| Providencia stuardii CNUR 5 | 2 | 8 | 32 | 128 | 2 | 16 | 1 | 8 | 4 |
| Klebsiella pneumoniae ATCC 10031 | 0.12 | 0.03 | 0.25 | 0.25 | 0.03 | 0.03 | 0.03 | 0.03 | 0.06 |
| Shighella enteritidis | 0.5 | 0.25 | 0.5 | 16 | 0.25 | 0.25 | 0.5 | 2 | 0.25 |
| Pseudomonas aeruginosa CNUR 2 | 32 | 16 | 8 | 128 | 8 | 4 | 16 | 128 | 8 |
| Pseudomonas aeruginosa CNUR 4 | 64 | 64 | 64 | 128 | 64 | 64 | 128 | 128 | 32 |
| Pseudomonas aeruginosa ATCC 9027 | 8 | 4 | 2 | 128 | 2 | 2 | 2 | 8 | 2 |
| Log | 0.118 | -0.065 | 0.099 | 1.124 | -0.125 | -0.168 | -0.126 | 0.317 | -0.106 |
| Geometrical Average | 1.312 | 0.861 | 1.256 | 13.30 | 0.750 | 0.680 | 0.748 | 2.078 | 0.783 |

(*) The product precipitates in the dish at 64 µg/ml.

TABLE N. 1 CONTINUATION - Minimum Inhibiting Concentration (µg/ml)

| MICROORGANISMS COMPOUND: | 23 | 28 | 33 | 34 | 35 | 36 | 40 | 42 | 44 |
|---|---|---|---|---|---|---|---|---|---|
| Gram + | | | | | | | | | |
| Staphylococcus aureus MPR 5 | 0.25 | 2 | 1 | 2 | 0.25 | 0.5 | 4 | 2 | 2 |
| Staphylococcus aureus ATCC 6538 | 0.5 | 2 | 0.5 | 1 | 0.25 | 0.5 | 4 | 2 | 2 |
| Staphylococcus epidermidis HCF Berset C. | 1 | 4 | 0.5 | 2 | 1 | 2 | 4 | 4 | 4 |
| Staphylococcus epidermidis CPHL A2 | 0.5 | 4 | 1 | 1 | 0.5 | 1 | 4 | 4 | 2 |
| Streptococcus facealis LEP Br. | 4 | 4 | 8 | 8 | 2 | 4 | 32 | 16 | 32 |
| Log | -0.120 | 0.482 | 0.060 | 0.301 | -0.241 | 0.060 | 0.783 | 0.602 | 0.602 |
| Geometrical Average | 0.758 | 3.031 | 1.149 | 2 | 0.574 | 1.149 | 6.063 | 4.00 | 4.00 |
| Gram - | | | | | | | | | |
| Escherichia coli CNUR 1 | 0.5 | 0.25 | 0.06 | 0.12 | 0.5 | 4 | 0.12 | 1 | 1 |
| Escherichia coli CNUR 8 | 0.25 | 0.12 | 0.06 | 0.06 | 0.25 | 2 | 0.06 | 0.25 | 0.5 |
| Escherichia coli 120 | 0.25 | 0.12 | 0.06 | 0.12 | 0.25 | 2 | 0.06 | 0.25 | 0.5 |
| Escherichia coli ATCC 8739 | 0.12 | 0.03 | 0.06 | 0.12 | 0.06 | 0.12 | 0.12 | 0.06 | 0.06 |
| Escherichia coli ISF 432 | 0.06 | 0.03 | 0.03 | 0.06 | 0.06 | 0.25 | 0.03 | 0.06 | 0.06 |
| Enterobacter cloacae OMNF 1153 | 1 | 0.25 | 0.25 | 0.25 | 0.5 | 8 | 0.12 | 1 | 2 |
| Enterobacter cloacae OMNF 1174 | 1 | 0.12 | 0.06 | 0.12 | 1 | 8 | 0.12 | 1 | 1 |
| Acinebacter calcoloaceticus OSMPV 1130 | 1 | 2 | 2 | 4 | 1 | 8 | 2 | 4 | 4 |
| Proteus vulgaris CNUR 6 | 64 | 64 | 8 | 8 | 64 | 64 | 64 | 64 | 128 |
| Providencia stuardii CNUR 5 | 0.5 | 2 | 2 | 2 | 1 | 2 | 2 | 4 | 4 |
| Klebsiella pneumoniae ATCC 10031 | 0.03 | 0.03 | 0.03 | 0.06 | 0.03 | 0.12 | 0.03 | 0.06 | 0.06 |
| Shighella enteritidis | 4 | 0.25 | 0.06 | 0.06 | 0.5 | 8 | 0.12 | 0.5 | 1 |
| Pseudomonas aeruginosa CNUR 2 | 8 | 8 | 8 | 16 | 8 | 8 | 4 | 16 | 32 |
| Pseudomonas aeruginosa CNUR 4 | 64 | 64 | 16 | 16 | 64 | 128 | 64 | 128 | 128 |
| Pseudomonas aeruginosa ATCC 9027 | 4 | 4 | 2 | 4 | 4 | 32 | 2 | 8 | 4 |
| Log | 0.016 | -0.228 | -0.471 | -0.290 | -0.064 | 0.600 | -0.332 | 0.137 | 0.237 |
| Geometrical Average | 1.037 | 0.592 | 0.338 | 0.51 | 0.863 | 3.978 | 0.466 | 1.371 | 1.727 |

TABLE N. 1 CONTINUATION - Minimum Inhibiting Concentration (μg/ml)

| MICROORGANISMS COMPOUND: | 46 | 47 | 48 | 50 | 51 | 52 |
|---|---|---|---|---|---|---|
| Gram + | | | | | | |
| Staphylococcus aureus MPR 5 | 0.25 | 0.5 | 1 | 4 | 0.5 | 1 |
| Staphylococcus aureus ATCC 6538 | 0.25 | 0.25 | 1 | 8 | 0.5 | 0.5 |
| Staphylococcus epidermidis HCF Berset C. | 0.5 | 1 | 2 | 8 | 2 | 4 |
| Staphylococcus epidermidis CPHL A2 | 0.5 | 0.5 | 1 | 8 | 2 | 2 |
| Streptococcus facealis LEP Br. | 8 | 8 | 16 | 64 | 64 | 64 |
| Log | -0.181 | -0.060 | 0.301 | 1.023 | 0.361 | 0.482 |
| Geometrical Average | 0.660 | 0.871 | 2.00 | 10.556 | 2.297 | 3.031 |
| Gram - | | | | | | |
| Escherichia coli CNUR 1 | 1 | 8 | 64 | 4 | 4 | 4 |
| Escherichia coli CNUR 8 | 0.25 | 2 | 16 | 0.5 | 0.5 | 1 |
| Escherichia coli 120 | 0.25 | 2 | 16 | 0.5 | 0.5 | 1 |
| Escherichia coli ATCC 8739 | 0.06 | 0.12 | 0.5 | 0.25 | 0.06 | 0.12 |
| Escherichia coli ISF 432 | 0.03 | 0.12 | 0.5 | 0.25 | 0.03 | 0.12 |
| Enterobacter cloacae OMNF 1153 | 2 | 16 | 128 | 4 | 4 | 8 |
| Enterobacter cloacae OMNF 1174 | 2 | 8 | 64 | 4 | 4 | 4 |
| Acinebacter calcoloaceticus OSMPV 1130 | 0.5 | 2 | 1 | 16 | 4 | 4 |
| Proteus vulgaris CNUR 6 | 128 | 128 | 128 | 128 | 128 | 128 |
| Providencia stuardii CNUR 5 | 4 | 4 | 128 | 4 | 4 | 8 |
| Klebsiella pneumoniae ATCC 10031 | 0.03 | 0.12 | 0.25 | 0.12 | 0.03 | 0.12 |
| Shighella enteritidis | 2 | 8 | 64 | 4 | 4 | 4 |
| Pseudomonas aeruginosa CNUR 2 | 16 | 64 | 128 | 32 | 128 | 128 |
| Pseudomonas aeruginosa CNUR 4 | 128 | 128 | 128 | 128 | 128 | 128 |
| Pseudomonas aeruginosa ATCC 9027 | 4 | 32 | 128 | 8 | 128 | 128 |
| Log | 0.117 | 0.679 | 1.284 | 0.541 | 0.478 | 0.659 |
| Geometrical Average | 1.309 | 4.773 | 19.248 | 3.473 | 3.007 | 4.557 |

## Claims

**1.** 6-aminoquinolones, having the following general formula (I):

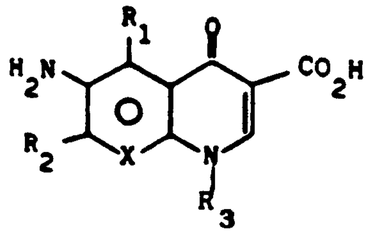

(I)

wherein

X = CH, $CCH_3$, CF, N;

$R_1$ = H, F, $NH_2$;

$R_2$ =

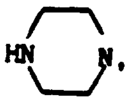,
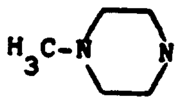,
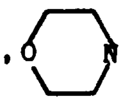,
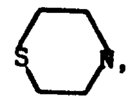,
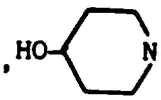

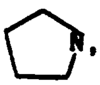,
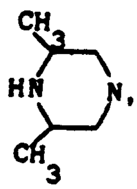,
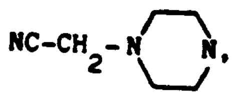,
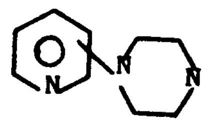

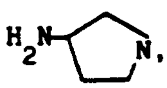,
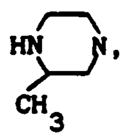,
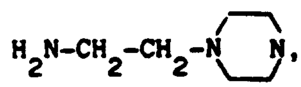,

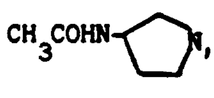,
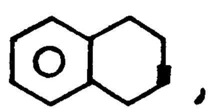,
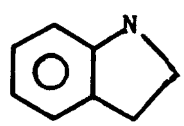

$R_3$ =

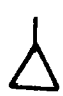, t-butyl, 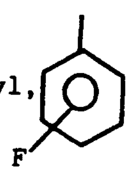

2. The use of the compounds according to claim 1 as antibacterial agents.

3. Antibacterial compositions comprising as active principle an effective amount of at least a compound according to claim 1.

European Patent Office

# EUROPEAN SEARCH REPORT

Application Number

EP 92 11 5387

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 107, 1987, Columbus, Ohio, US; abstract no. 96604b, MASUZAWA, KUNIYASU ET AL. 'Preparation of quinolonecarboxylic acid derivatives as bactericides.' page 680 ; * abstract * RN 109887-89-0, RN 109887-88-9, RN 109887-76-5, RN 109887-75-4, RN 109887-74-3, RN 109887-73-2 * & JP 8726272 (kyorin pharmaceutical co. ) --- | 1-3 | C07D215/56 A61K31/40 C07D401/04 C07D471/04 C07D401/12 |
| X | JOURNAL OF MEDICINAL CHEMISTRY. vol. 27, no. 3, 1984, WASHINGTON US pages 292 - 301 JUN-ICHI MATSUMOTO ET AL. 'Pyridonecarboxylic acids as antibacterial agents. 2.' * page 294, compounds 23 a-c * --- | 1-3 | |
| A | JOURNAL OF MEDICINAL CHEMISTRY. vol. 33, no. 5, 1990, WASHINGTON US pages 1344 - 1352 D. BOUZARD ET AL. 'Fluoronaphthyridines and quinolones as antibacterial agents.2.' * table 1 * --- | 1-3 | TECHNICAL FIELDS SEARCHED (Int. Cl.5) C07D |
| A | JOURNAL OF MEDICINAL CHEMISTRY. vol. 33, no. 1, 1990, WASHINGTON US pages 142 - 146 C.B. ZIEGLER ET AL. 'Synthesis and structure-activity relationships of new 7-(3-(fluoromethyl)piperazinyl)- and -(fluorohomopiperazinyl)quinolone antibacterials.' * table 1 * --- -/-- | 1-3 | |

The present search report has been drawn up for all claims

| Place of search | Date of completion of the search | Examiner |
|---|---|---|
| THE HAGUE | 27 OCTOBER 1992 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 92 11 5387
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | CHEMICAL ABSTRACTS, vol. 117 Columbus, Ohio, US; abstract no. 292f, YAMASHITA,YOSHINORI ET AL 'Antitumour quinolones with mammalian topoisomerase II mediated DNA cleavage activity.' page 290 ; * abstract * * RN 141945-91-7 * & Cancer Res. 1992,52(10),2818-22. | 1 | |
| | ----- | | TECHNICAL FIELDS SEARCHED (Int. Cl.5 ) |

The present search report has been drawn up for all claims

| Place of search | Date of completion of the search | Examiner |
|---|---|---|
| THE HAGUE | 27 OCTOBER 1992 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document